# EUROPÄISCHE PATENTSCHRIFT

(11) **EP 0 387 747 B1**
(45) Veröffentlichungstag und Bekanntmachung des Hinweises auf die Patenterteilung: **17.05.1995**
(21) Anmeldenummer: 90104620.1
(22) Anmeldetag: 12.03.1990
(51) Int. Cl.: C07D 495/04, C07F 9/6561

(54) **Verfahren zur Herstellung von 1,3-substituiertem Tetrahydro-1H-thieno-[3,4-d]-imidazol-2(3H)-on-4-yliden-pentansäureester**
Process for the preparation of 1,3-substituted tetrahydro-1H-thieno[3,4-d]imidazol-2(3H)-on-4-ylidene-pentanoic ester
Procédé pour la préparation de l'ester tétrahydro-1H-thiéno[3,4-d]imidazol-2(3H)-on-4-ylidène-pentanoique 1,3-substitué

(30) Priorität: 15.03.1989 CH 953/89
(43) Veröffentlichungstag der Anmeldung: 19.09.1990
(73) Patentinhaber: LONZA AG, CH-3945 Gampel/Wallis (CH)
(72) Erfinder: Eyer, Martin, Dr., Glis (Wallis) (CH)
(74) Vertreter: Weinhold, Peter, Dr.

(56) Entgegenhaltungen:
- EP-A- 0 084 377
- EP-A- 0 270 076
- EP-A- 0 273 270
- DE-A- 3 018 109
- JOURNAL OF ORGANIC CHEMISTRY Band 44, Nr. 16, 1979, Seiten 2911-2915; C.G. KRUSE et al.: "Reactions of 2-Chlorotetrahydrofuran and 2-Chlorotetrahydrothiophene with Phosphorus, Carbon, and Nitrogen Nucleophiles"

## Beschreibung

Die Erfindung beinhaltet ein Verfahren zur Herstellung der Titelverbindungen als geeignete Vorstufen zur Herstellung von (+)-Biotin.

(+)-Biotin ist als menschliches Vitamin, als Vitamin H, bekannt. Biotin wird aber auch als pharmazeutische Wirksubstanz zur Behandlung von Dermatose und als Futtermittelzusatz mit wachstumssteigernder Wirkung für Nutztiere eingesetzt.

Die DE-A-30 18 109 offenbart ein Verfahren zur Herstellung von Biotin, in dem man zunächst 2-Formyl-3,4-diamino-thiophenderivate mit üblichen abspaltbaren Aminoschutzgruppen mit einem Wittig-Reagens zu den entsprechenden 5-(3,4-Diamino-thienyl)-pentensäure- oder -pentadiensäurederivaten reagieren läßt, und diese anschließend nach Hydrierung und Abspaltung der Schutzgruppen in einer Carbonylierungsreaktion zum gewünschten Endprodukt umsetzt.

Die EP-A-0 084 377 offenbart ein Verfahren zur Herstellung von Biotin oder seinen Estern, wobei in einer ersten Stufe 1,3-substituierte Tetrahydro-1H-thieno-[3,4-d]-imidazol-2,4-dione mit einem Wittig-Reagens zu den entsprechenden 1,3-substituierten Tetrahydro-1H-thieno-[3,4-d]-imidazol-2H-on-yliden-pentansäureestern umgesetzt werden.

Aus der EP-A-0 270 076 und der EP-A-0 273 270 ist als Zwischenstufe in der (+)-Biotinsynthese bekannt, 1,3-substituierte Tetrahydro-1H-thieno-[3,4-d]-imidazol-2(3H)-on-4-yliden-pentansäure aus dem 1,3-substituierten Tetrahydro-1H-thieno-[3,4-d]-imidazol-2,4-dion mittels einer Grignard-Reaktion oder mittels Wittig-Reaktion herzustellen. Von großem Nachteil sind aber die sehr schlechten Ausbeuten von 12% bei der Wittig-Reaktion bzw. 28% bei der Umsetzung nach Grignard.

Es bestand daher die Aufgabe, diese Stufe in der Biotinsynthese in Bezug auf die Ausbeute deutlich zu verbessern. Diese Aufgabe konnte gelöst werden mit einem Verfahren nach Patentanspruch 1, worin man 1,3-substituierte Tetrahydro-1H-thieno-[3,4-d]-imidazol-2,4-dione der Formel 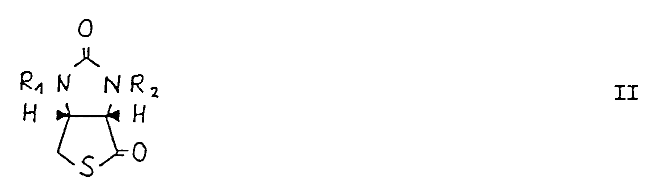
worin R₁ eine (R)- oder (S)-1-Phenylalkylgruppe, eine (R)- oder (S)-1-Alkoxycarbonyl- 1-phenylmethylgruppe oder eine (R)- oder (S)-1-Aryloxycarbonyl-1-phenylmethylgruppe und R₂ Wasserstoff, eine Alkanoylgruppe substituiert oder unsubstituiert, eine Benzoylgruppe bzw. eine Benzylgruppe, jeweils unsubstituiert oder substituiert, eine Alkoxycarbonylgruppe, eine Aryloxycarbonylgruppe, eine Aryloxyalkylgruppe, eine Alkoxyalkylgruppe, eine Pyranylgruppe, eine Benzolsulfonylgruppe unsubstituiert oder substituiert, eine Alkylsulfonylgruppe, eine Diarylphosphinylgruppe, eine Dialkoxyphosphinylgruppe oder eine Trialkylsilylgruppe bedeuten, mit einem Reduktionsmittel in Gegenwart eines inerten Lösungsmittels zur entsprechenden Hydroxyverbindung reduziert, diese mit einem Phosphoniumsalz der Formel

(R₃)₃PH^{⊕} X^{⊖} III

worin R₃ eine Alkylgruppe mit 1 bis 20 C-Atomen, eine Aryl- oder Benzylgruppe, und X ein Halogenatom, BF₄⁻, ClO₄⁻, J₃⁻ oder PF₆⁻ bedeuten, in Gegenwart eines inerten Lösungsmittels in eine Phosphoniumverbindung der Formel 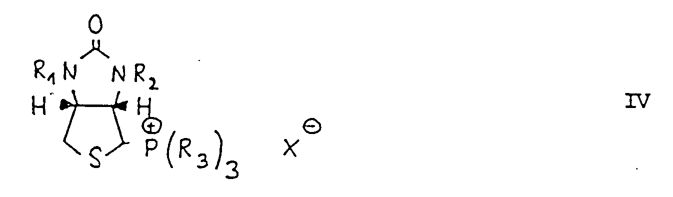
worin R₁, R₂, R₃ und X die genannte Bedeutung haben, umsetzt und schliesslich in Gegenwart einer starken Base und eines inerten Lösungsmittels mit einem 5-Oxo-valeriansäureester der Formel 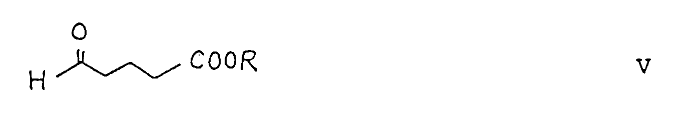
worin R = C₁-C₆ Alkyl geradkettig oder verzweigt, substituiert oder unsubstituiert, oder eine Phenyl- oder Benzylgruppe unsubstituiert oder substituiert, mit Halogenatomen oder Alkyl- oder Arylgruppen bedeutet, in den 1,3-substituierten Tetrahydro-1H-thieno-[3,4-d]-imidazol-2(3H)-on-4-yliden-pentansäureester der Formel 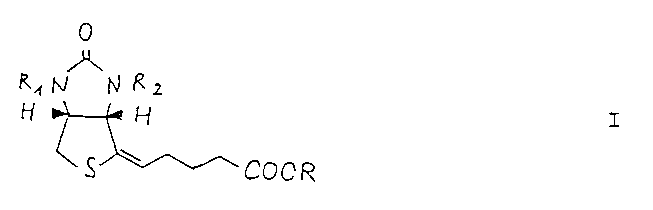
worin R, R₁ und R₂ die genannte Bedeutung hat, überführt.

Zweckmässig wird unter R eine Methyl-, Ethyl-, Propyl-, i-Propyl-, Butyl-, Phenyl- oder Benzylgruppe verstanden.

R₁ steht zweckmäßig für eine (R)- oder (S)-1-phenyl-(C₂-C₄)-alkylgruppe, eine (R)- oder (S)-1-(C₁-C₄)-alkoxycarbonyl-1-phenylmethylgruppe, eine (R)- oder (S)-1-Benzyloxycarbonyl-1-phenylmethylgruppe oder eine (R)- oder (S)-1-Phenyloxycarbonyl-1-phenylmethylgruppe. Besonders bevorzugt wird die (R)- oder (S)-1-Phenylethylgruppe.

R₂ steht zweckmäßig für Wasserstoff; für eine (C₁-C₄)-Alkanoylgruppe, die z.B. durch Halogenatome substituiert sein kann (hiervon wird die Trichloracetylgruppe bevorzugt), für eine unsubstituierte oder durch Halogenatome bzw.(C₁-C₆)-Alkyl- bzw.(C₁-C₆)-Alkoxygruppen substituierte Benzoyl- oder Benzylgruppe, eine (C₁-C₄)-Alkoxycarbonylgruppe, eine Phenyloxycarbonylgruppe, eine (C₁-C₄)-Alkoxymethylgruppe oder eine p-Toluolsulfonyl- bzw. Methansulfonyl-Gruppe.

Generell wird unter dem Begriff Alkyl bzw. Alkoxy bevorzugt (C₁-C₄)-Alkyl bzw. (C₁-C₄)-Alkoxy verstanden und unter dem Begriff Aryl bevorzugt unsubstituiertes Benzyl oder Phenyl.

Besonders bevorzugt bedeutet R₂ Wasserstoff oder eine Acetyl-, Benzyl-, 4-Methoxybenzyl-, tert.-Butoxycarbonyl- oder Methoxymethylgruppe.

Geeignete Reste R₃ sind (C₁-C₄)-Alkyl, Benzyl oder Phenyl, besonders geeignet ist Phenyl.

Die Herstellung der 1,3-substituierten Tetrahydro-1H-thieno-[3,4-d]-imidazol-2,4-dione als Ausgangsverbindungen ist aus EP-Anm. 273 270 bekannt.

Zur Reduktion von II werden zweckmässig Diisobutylaluminiumhydrid, Natriumborhydrid, Borankomplexe oder komplexe Aluminiumhydride verwendet.
Vorzugsweise wird Diisobutylaluminiumhydrid bei Temperaturen zwischen -80 und 25°C eingesetzt.
Das Reduktionsmittel wird zweckmässig in einem Ueberschuss von 5 bis 20%, bezogen auf das Ausgangsprodukt, zugesetzt.
Die Reduktion erfolgt in Gegenwart eines inerten Lösungsmittels, wie z.B. Toluol, Tetrahydrofuran, Ether, Benzol oder Dimethoxyethan.

Nach einer Reaktionszeit von üblicherweise 15 bis 120 min kann das resultierende 1,3-substituierte 4-Hydroxy-1H-thieno-[3,4-d]-imidazol-2(3H)-on auf übliche Weise isoliert werden.

Die Ausbeute in dieser ersten Stufe ist in der Regel nahezu quantitativ.

Die Folgestufe umfasst die Bildung der Phosphoniumverbindung der allgemeinen Formel 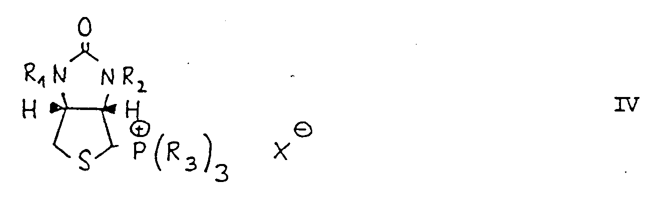
mit Hilfe eines Phosphoniumsalzes der Formel

(R₃)₃PH^{⊕} X^{⊖} III

worin die Substituenten R₃ und X die genannte Bedeutung haben.

Die Verbindungen IV sind bisher nicht vorbeschrieben. Als neue Zwischenprodukte sind sie wesentlicher Bestandteil dieser Erfindung.

Besonders geeignete Phosphoniumsalze III sind Triphenylphosphonium-tetrafluoroborat oder Triphenylphosphoniumchlorid. Die Phosphoniumsalze III werden in der Regel kurz vor ihrem Einsatz auf bekannte Weise, z.B. nach D.A.Clark et al, Synthesis 1977, 628, hergestellt.

Die Umsetzung erfolgt abhängig vom Lösungsmittel zweckmässig bei Temperaturen von -20 bis 100°C, vorteilhaft bei 60 bis 80°C, in einem inerten Lösungsmittel.

Geeignete Lösungsmittel sind z.B. Acetonitril, Tetrahydrofuran, Ether, Dioxan oder Dimethoxyethan, vorzugsweise Acetonitril.

Nach einer Reaktionszeit von 30 bis 300 min kann die Verbindung IV in nahezu quantitativer Ausbeute auf fachmännische Art und Weise isoliert und gegebenenfalls gereinigt werden.

Die letzte Stufe beinhaltet die Ueberführung der Phosphoniumverbindung IV in Gegenwart einer starken Base mit einem 5-Oxo-valeriansäureester der Formel V zum Endprodukt.

Als starke Basen können die Alkalialkoholate von Alkoholen mit 1-4 C-Atomen, wie z.B. Kalium-tert.butylat oder Natriummethylat, oder Alkyllithium wie z.B. n-Butyllithium, oder ein Alkalihexamethyldisilazid wie z.B. Natriumhexamethyldisilazid, oder ein Alkalihydrid wie z.B. Natriumhydrid eingesetzt werden. Besonders geeignet ist Kalium-tert.butylat oder Natriumhexamethyldisilazid.
Zweckmässig wird die Base in einem Ueberschuss von 5 bis 20%, vorzugsweise von 10 bis 15%, eingesetzt.
Die Reaktionstemperatur wird vorteilhaft zwischen -20 und 60°C, zweckmässig zwischen 0 und 25°C, gehalten.

Im weiteren ist es zweckmässig, in Gegenwart von inerten Lösungsmitteln, wie z.B. Tetrahydrofuran, Dimethylsulfoxid, Ether, zu arbeiten.

Nach einer Reaktionszeit von 2 bis 24 h kann das Endprodukt auf übliche Weise in Ausbeuten von gegen 70% aus dem Reaktionsgemisch isoliert und gegebenenfalls gereinigt werden.

Damit lässt sich mit dem erfindungsgemässen 3-Stufen-Verfahren eine Gesamtausbeute von über 60% realisieren.

### Beispiel

### a) Herstellung von (3aS,6aR)-3-Benzyl-1-[(R)-(1-phenylethyl)]-tetrahydro-4-hydroxy-1H-thieno-[3,4-d]-imidazol-2(3H)-on

Zu einer auf -70°C abgekühlten Lösung von 3,52 g (10 mmol) (3aS,6aR)-3-Benzyl-1-[(R)-(1-phenylethyl)]-tetrahydro-1H-thieno-[3,4-d]-imidazol-2,4-dion in 120 ml Toluol wurden unter Rühren langsam 10 ml einer 1,2 M Lösung von Diisobutylaluminiumhydrid in Toluol getropft, wobei die Temperatur der Reaktionslösung nicht über -60°C stieg.
Nach beendeter Zugabe wurde 2 h bei -70°C gerührt und 50 ml 10%ige wässrige Ammoniunchloridlösung zugegeben. Das teilweise ausgefallene weisse Produkt wurde abfiltriert, die Phasen getrennt und die Wasserphase noch zweimal mit Essigester extrahiert. Die vereinigten organischen Phasen wurden mit MgSO₄ getrocknet und am Rotavapor eingeengt.
Der weisse Rückstand wurde mit dem bereits abfiltrierten Produkt vereinigt.
Total wurden 3,49 g (98,6%) (3aS,6aR)-3-Benzyl-1-[(R)-(1-phenylethyl)]-tetrahydro-4-hydroxy-1H-thieno-[3,4-d]-imidazol-2(3H)-on als (94:6)-Diastereomergemisch erhalten. Die nachfolgenden spektroskopischen Daten sind vom Hauptdiastereoisomer.
- Smp: 216-217°C
- IR (KBr): 3235, 2930, 1668, 1470, 1245, 1040, 754
- ¹H-NMR (d₆-DMSO): 1,58 (d,J = 7,5 Hz, 3H)
2,25 (d,J = 12,5 Hz, 1H)
2,85 (dd,J = 5 Hz, 12,5 Hz, 1H)
3,90 (d,J = 8,7 Hz, 1H)
4,25 (d,J = 15 Hz, 1H)
4,55 (m, 1H)
4,60 (d,J = 15 Hz, 1H)
5,05 (q,J = 7,5 Hz, 1H)
5,22 (d,J = 4,5 Hz, 1H)
6,00 (d,J = 4,5 Hz, 1H)
7,20-7,50 (m, 10H)
- MS (m/e) 354 (M⁺,2): 187 (28), 105 (66), 97 (16), 91 (100), 77 (14), 44 (19), 36 (38)

### b) Herstellung von (3aS,6aR)-3-Benzyl-1-[(R)-(1-phenylethyl)]-tetrahydro-4-triphenylphosphonium-1H-thieno-[3,4-d]-imidazol-2(3H)-on-tetrafluoroborat

Eine Lösung von 0,70 g (2 mmol) (3aS,6aR)-3-Benzyl-1-[(R)-(1-phenylethyl)]-tetrahydro-4-hydroxy-1H-thieno-[3,4-d]-imidazol-2(3H)-on und 0,77 g (2,2 mmol) Triphenylphosphonium-tetrafluoroborat in 20 ml Acetonitril wurde während 2 h am Rückfluss gekocht. Kontrolle mittels DC zeigte, dass kein Edukt mehr vorhanden war. Das Lösungsmittel wurde am Rotavapor abdestilliert und der Rückstand getrocknet.
Ausbeute 1,35 g (98,5%) (3aS,6aR)-3-Benzyl-1-[(R)-(1-phenylethyl)]-tetrahydro-4-triphenylphosphonio-1H-thieno-[3,4-d]-imidazol-2(3H)-on-tetrafluoroborat.
- Smp: 116-118°C
- IR (KBr): 2935, 1697, 1479, 1450, 1439, 1427, 1252, 1107, 1071 1036, 996, 738, 689
- ¹H-NMR (CDCl₃): 1,15 (dd,J = 5,0 Hz, 12,5 Hz, 1H)
1,55 (d,J = 7,5 Hz, 3H)
1,85 (d,J = 12,5 Hz, 1H)
4,28 (d,J = 17,5 Hz, 1H)
4,40-4,50 (m, 1H)
4,80-4,90 (m, 1H)
4,90 (d,J = 17,5 Hz, 1H)
5,05 (t,J = 7,5 Hz, 1H)
5,28 (q,J = 7,5 Hz, 1H)
7,15-7,50 (m, 15H)
7,55-7,85 (m, 10H)

### c1) Herstellung von (3aS,6aR)-3-Benzyl-1-[(R)-(1-phenylethyl)]-tetrahydro-1H-thieno-[3,4-d]-imidazol-2(3H)-on-4-ylidenpentansäure-methylester

Zu einer Lösung von 2,28 g (3,30 mmol) (3aS,6aR)-3-Benzyl-1-[(R)-(1-phenylethyl)]-tetrahydro-4-triphenylphosphonio-1H-thieno-[3,4-d]-imidazol-2(3H)-on-tetrafluoroborat in 30 ml Tetrahydrofuran wurde bei Raumtemperatur portionenweise 0,44 g (3,80 mmol) frisch sublimiertes Kalium-tert.butylat gegeben. Das orangerote Reaktionsgemisch wurde 2 h bei Raumtemperatur gerührt und anschliessend 0,53 g (3,80 mmol) 5-Oxo-valeriansäuremethylester, verdünnt mit 1 ml Tetrahydrofuran, langsam zugetropft.
Das Reaktionsgemisch wurde über Nacht gerührt, in Wasser/Essigsäureethylester aufgenommen und das Lösungsmittel am Rotavapor eingeengt.
Kieselgel-Chromatographie mit Essigester/Hexan als Eluiermittel lieferte 0,97 g (65%) (3aS,6aR)-3-Benzyl-1-[(R)-(1-phenylethyl)]-tetrahydro-1H-thieno-[3,4-d]-imidazol-2(3H)-on-4-yliden-pentansäuremethylester als farbloses Oel.
- IR (Film): 2935, 1736, 1694, 1435, 1415, 1360, 1245, 1216, 1168, 756, 702
- ¹H-NMR (CDCl₃): 1,45-1,60 (m, 2H)
1,68 (d,J = 7,5 Hz, 3H)
1,75-1,88 (m, 2H)
2,08 (t,J = 7,5 Hz, 2H)
2,28 (dd,J = 3 Hz, 12,5 Hz, 1H)
2,52 (dd,J = 5,5 Hz, 12,5 Hz, 1H)
3,60 (s, 3H)
4,02 (d,J = 16,0 Hz, 1H)
4,33-4,41 (m, 1H)
4,65 (d,J = 7,5 Hz, 1H)
4,92 (d,J = 16,0 Hz, 1H)
5,38 (q,J = 7,5 Hz, 1H)
5,60 (t,J = 7,5 Hz, 1H)
7,15-7,50 (m, 10H)
- MS (m/e) 450 (M⁺,1): 345 (5), 237 (14), 120 (20), 105 (80), 91 (100), 79 (14), 44 (10)

### c2) Herstellung von (3aS,6aR)-3-Benzyl-1-[(R)-(1-phenylethyl)]-tetrahydro-1H-thieno-[3,4-d]-imidazol-2(3H)-on-4-yliden-pentansäure-hexylester

Zu einer Lösung von 2,50 g (3,64 mmol) (3aS,6aR)-3-Benzyl-1-[(R)-(1-phenylethyl)]-tetrahydro-4-triphenylphosphino-1H-thieno[3,4-d]imidazol-2(3H)-on-tetrafluoroborat in 35 ml Tetrahydrofuran wurde bei Raumtemperatur portionenweise 0,47 g (4,19 mmol) frisch sublimiertes Kalium-tert.butylat gegeben. Das orangerote Reaktionsgemisch wurde 2 h bei Raumtemperatur gerührt und anschliessend 0,84 g (4,20 mmol) 5-Oxo-valeriansäurehexylester, verdünnt mit 1 ml Tetrahydrofuran, langsam zugetropft. Das Reaktionsgemisch wurde über Nacht gerührt, in Wasser/Essigester aufgenommen und das Lösungsmittel am Rotavapor entfernt.
Kieselgel-Chromatographie mit Essigester/Hexan als Eluiermittel lieferte 1,10 g (58%)
(3aS,6aR)-3-Benzyl-1-[(R)-(1-phenylethyl)]-tetrahydro-1H-thieno-[3,4-d]-imidazol-2(3H)-on-4-yliden-pentansäurehexylester als schwach gelbliches Oel.
- IR (Film): 2931, 1732, 1696, 1432, 1413, 1359, 1244, 1216, 1169, 756, 701
- ¹H-NMR (CDCl₃): 0,85-1,00 (m, 3H)
1,25-1,45 (m, 6H)
1,55-1,80 (m, 7H)
1,95-2,15 (m, 2H)
2,27 (t,J = 7,5 Hz, 2H)
2,38 (dd,J = 3 Hz, 12,5 Hz, 1H)
2,49 (dd,J = 5,5 Hz, 12,5 Hz, 1H)
4,00-4,10 (m, 3H)
4,20-4,30 (m, 2H)
4,93 (d,J = 15,5 Hz, 1H)
5,32-5,45 (m, 2H)
7,20-7,50 (m, 10H)
- MS (m/e) 520 (M⁺,15),: 487 (10), 415 (32), 253 (14), 237 (41), 187 (11), 132 (17), 120 (21), 105 (100), 91 (92), 43 (28)

### c3) Herstellung von (3aS,6aR)-3-Benzyl-1-[(R)-(1-phenylethyl)]-tetrahydro-1H-thieno-[3,4-d]-imidazol-2(3H)-on-4-yliden-pentansäure-benzylester

Zu einer Lösung von 2,85 g (4,15 mmol) (3aS,6aR)-3-Benzyl-1-[(R)-(1-phenylethyl)]-tetrahydro-4-triphenylphosphonio-1H-thieno[3,4-d]imidazol-2(3H)-on-tetrafluoroborat in 35 ml Tetrahydrofuran wurde bei Raumtemperatur portionenweise 0,54 g (4,78 mmol) frisch sublimiertes Kalium-tert.butylat gegeben. Das orangerote Reaktionsgemisch wurde 2 h bei Raumtemperatur gerührt und anschliessend 0,99 g (4,78 mmol) 5-Oxo-valeriansäurebenzylester, verdünnt mit 1 ml Tetrahydrofuran, langsam zugetropft. Das Reaktionsgemisch wurde über Nacht gerührt, in Wasser/Essigester aufgenommen und das Lösungsmittel am Rotavapor ein geengt.
Kieselgel-Chromatographie mit Essigester/Hexan als Eluiermittel lieferte 1,57 g (72%) (3aS,6aR)-3-Benzyl-1-[(R)-(1-phenylethyl)]-tetrahydro-1H-thieno[3,4-d]imidazol-2(3H)-on-4-yliden-4-pentansäurebenzylester als farbloses Oel.
- IR (Film): 2935, 1734, 1694, 1444, 1414, 1358, 1236, 1215, 1161, 752, 700
- ¹H-NMR (CDCl₃): 1,62-1,78 (m, 5H)
1,98-2,15 (m, 2H)
2,33 (t,J = 7,5 Hz, 2H)
2,38 (dd,J = 4 Hz, 12,5 Hz, 1H)
2,46 (dd,J = 5,5 Hz, 12,5 Hz, 1H)
4,03 (d,J = 15,5 Hz, 1H)
4,18-4,28 (m, 2H)
4,93 (d,J = 15,5 Hz, 1H)
5,11 (s, 2H)
5,33-5,42 (m, 2H)
7,20-7,50 (m, 15H)
- MS (m/e) 526 (M⁺,14),: 493 (10), 435 (12), 421 (20), 331 (10), 253 (10), 237 (31), 187 (17), 132 (10), 105 (65), 91 (100), 79 (8), 65 (4)

## Patentansprüche

1. Verfahren zur Herstellung von 1,3-substituiertem Tetrahydro-1H-thieno-[3,4-d]-imidazol-2(3H)-on-4-yliden-pentansäureester der Formel 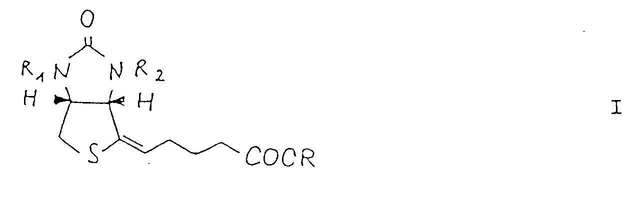 worin R = C₁-C₆ Alkyl geradkettig oder verzweigt, unsubstituiert oder substituiert, oder eine Phenyl- oder Benzylgruppe unsubstituiert oder substituiert, mit Halogenatomen oder Alkyl- oder Arylgruppen bedeutet, und R₁ eine (R)- oder (S)-1-Phenylalkylgruppe, eine (R)- oder (S)-1-Alkoxycarbonyl-1-phenylmethylgruppe oder eine (R)-oder (S)-1-Aryloxycarbonyl-1-phenylmethylgruppe und R₂ Wasserstoff, eine Alkanoylgruppe substituiert oder unsubstituiert, eine Benzoylgruppe bzw. eine Benzylgruppe, jeweils unsubstituiert oder substituiert, eine Alkoxycarbonylgruppe, eine Aryloxycarbonylgruppe, eine Aryloxyalkylgruppe, eine Alkoxyalkylgruppe, eine Pyranylgruppe, eine Benzolsulfonylgruppe, unsubstituiert oder substituiert, eine Alkylsulfonylgruppe, eine Diarylphosphinylgruppe, eine Dialkoxyphosphinylgruppe oder eine Trialkylsilylgruppe bedeuten, dadurch gekennzeichnet, daß man ein 1,3-substituiertes Tetrahydro-1H-thieno-[3,4-d]-imidazol-2,4-dion der Formel 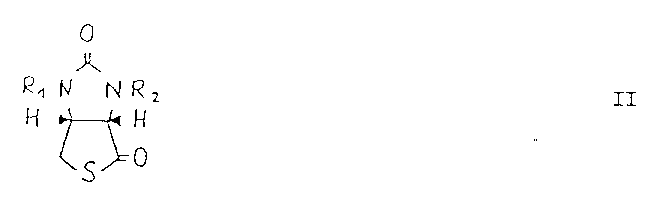 worin R₁ und R₂ die genannte Bedeutung haben, mit einem Reduktionsmittel in Gegenwart eines inerten Lösungsmittels zur entsprechenden Hydroxyverbindung reduziert, diese mit einem Phosphoniumsalz der Formel
(R₃)₃PH^{⊕} X^{⊖} III
worin R₃ eine Alkylgruppe mit 1 bis 20 C-Atomen, eine Aryl- oder Benzylgruppe, und X ein Halogenatom, BF₄⁻, ClO₄⁻, J₃⁻ oder PF₆⁻ bedeuten, in Gegenwart eines inerten Lösungsmittels in eine Phosphoniumverbindung der Formel 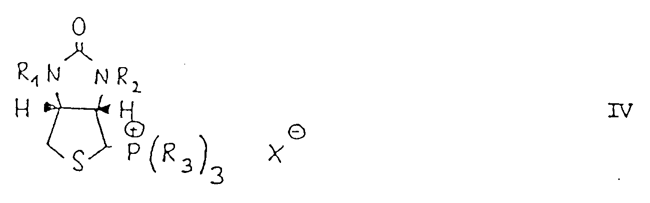 worin R₁, R₂, R₃ und X die genannte Bedeutung haben, umsetzt und schließlich in Gegenwart einer starken Base und in einem inerten Lösungsmittel mit einem 5-Oxo-valeriansäureester der Formel 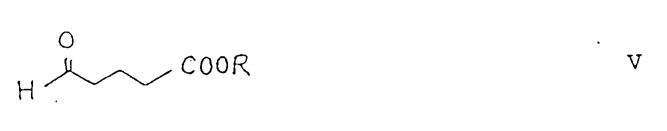 worin R die genannte Bedeutung hat, in das Endprodukt überführt.

2. Verfahren nach Patentanspruch 1, dadurch gekennzeichnet, daß als Reduktionsmittel Diisobutylaluminiumhydrid, Natriumborhydrid, Borankomplexe oder komplexe Aluminiumhydride verwendet werden.

3. Verfahren nach Patentanspruch 1, dadurch gekennzeichnet, daß man die Reduktion mit Diisobutylaluminiumhydrid bei Temperaturen von -80 bis 25°C durchführt.

4. Verfahren nach mindestens einem der Patentansprüche 1 bis 3, dadurch gekennzeichnet, daß die Bildung der Phosphoniumverbindung bei Temperaturen von -20 bis 100°C erfolgt.

5. Verfahren nach mindestens einem der Patentansprüche 1 bis 4, dadurch gekennzeichnet, daß als starke Base für die Umsetzung mit dem 5-Oxo-valeriansäureester ein Alkalialkoholat, ein Alkalihexamethyldisilazid, ein Alkalihydrid oder ein Alkyllithium eingesetzt wird.

6. Verfahren nach mindestens einem der Patentansprüche 1 bis 5, dadurch gekennzeichnet, daß die Umsetzung mit dem 5-Oxo-valeriansäureester bei Temperaturen von -20 bis 60°C erfolgt.

7. Phosphoniumverbindungen als Zwischenprodukte im Verfahren nach Patentanspruch 1 der Formel 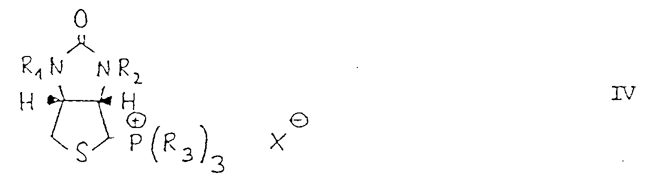 worin R₁ eine (R)- oder (S)-1-Phenyl-(C₂-C₄)-alkylgruppe, eine (R)- oder (S)-1-(C₁-C₄)-Alkoxycarbonyl-1-phenylmethylgruppe, eine (R)- oder (S)-1-Benzyloxycarbonyl-1-phenylmethylgruppe oder eine (R)- oder (S)-1-Phenyloxycarbonyl-1-phenylmethylgruppe bedeutet; R₂ Wasserstoff, eine unsubstituierte oder durch Halogenatome substituierte (C₁-C₄)-Alkanoylgruppe, eine unsubstituierte oder durch Halogenatome bzw. (C₁-C₆)-Alkyl- bzw. (C₁-C₆)-Alkoxygruppen substituierte Benzoyl- oder Benzylgruppe, eine (C₁-C₄)-Alkoxycarbonylgruppe, eine Phenyl- oder Benzyloxycarbonylgruppe, eine (C₁-C₄)-Alkoxymethylgruppe, eine Pyranylgruppe, eine Benzolsulfonylgruppe, eine p-Toluolsulfonylgruppe, eine (C₁-C₄)-Alkylsulfonylgruppe, eine Diphenyl- oder Dibenzylphosphinylgruppe, eine Di-(C₁-C₄)-alkoxyphosphinylgruppe oder eine Tri-(C₁-C₄)-alkylsilylgruppe bedeutet; R₃ eine Alkylgruppe mit 1 - 20 C-Atomen, eine Phenyl- oder Benzylgruppe bedeutet; und X ein Halogenatom, BF₄⁻, ClO₄⁻, J₃⁻ oder PF₆⁻ bedeutet.

## Claims

1. Process for the preparation of a 1,3-substituted tetrahydro-1H-thieno-[3,4-d]-imidazol-2(3H)-on-4-ylidene-pentanoic acid ester of formula 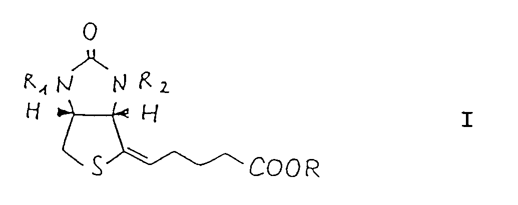 wherein R = straight-chain or branched C₁-C₆ alkyl, unsubstituted or substituted, or represents a phenyl or benzyl group, unsubstituted or substituted by halogen atoms or alkyl or aryl groups, and R₁ represents an (R)- or (S)-1-phenylalkyl group, an (R)- or (S)-1-alkoxycarbonyl-1-phenylmethyl group, or an (R)- or (S)-1-aryloxycarbonyl-1-phenylmethyl group, and R₂ represents hydrogen, an unsubstituted or substituted alkanoyl group, a benzoyl group or benzyl group, respectively, each unsubstituted or substituted, an alkoxycarbonyl group, an aryloxycarbonyl group, an aryloxyalkyl group, an alkoxyalkyl group, a pyranyl group, a benzenesulfonyl group, unsubstituted or substituted, an alkylsulfonyl group, a diarylphosphinyl group, a dialkoxyphosphinyl group or a trialkylsilyl group, characterized in that a 1,3-substituted tetrahydro-1H-thieno-[3,4-d]-imidazol-2,4-dione of formula 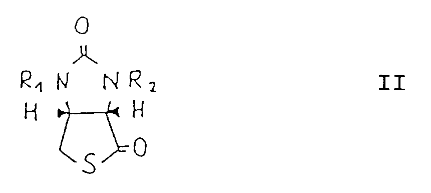 wherein R₁ and R₂ are as defined above, is reduced, in the presence of an inert solvent, to the corresponding hydroxy compound using a reducing agent, that said hydroxy compound is reacted, in the presence of an inert solvent, with a phosphonium salt of formula
(R₃)₃PH^{⊕} X^{⊖} III
wherein R₃ represents an alkyl group having 1 to 20 C-atoms, an aryl or benzyl group, and X represents a halogen atom, BF₄⁻, ClO₄⁻, J₃⁻, or PF₆⁻, to form a phosphonium compound of formula 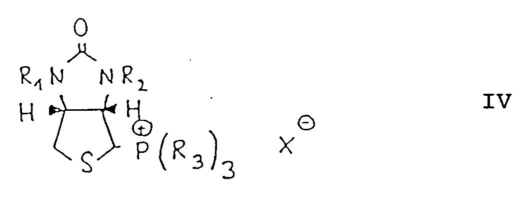 wherein R₁, R₂, R₃, and X are as defined above, which subsequently is converted, in the presence of a strong base and in an inert solvent, to the final product with a 5-oxo-valeric acid ester of formula 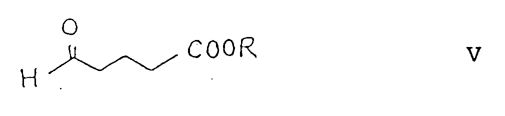 wherein R is as defined above.

2. Process according to claim 1, characterized in that diisobutylaluminum hydrid, sodium borohydrid, borane complexes, or complex aluminum hydrids are used as reducing agents.

3. Process according to claim 1, characterized in that the reduction is carried out at temperatures of from -80 to 25°C using diisobutylaluminum hydrid.

4. Process according to at least one of claims 1 to 3, characterized in that formation of the phosphonium compound is effected at temperatures of from -20 to 100°C.

5. Process according to at least one of claims 1 to 4, characterized in that as the strong base in the reaction with the 5-oxo-valeric acid ester an alkali alkoholate, an alkali hexamethyldisilazide, an alkali hydrid, or an alkyl lithium is used.

6. Process according to at least one of claims 1 to 5, characterized in that the reaction with the 5-oxo-valeric acid ester is performed at temperatures of from -20 to 60°C.

7. Phosphonium compounds as intermediate products in a process according to claim 1 having the formula 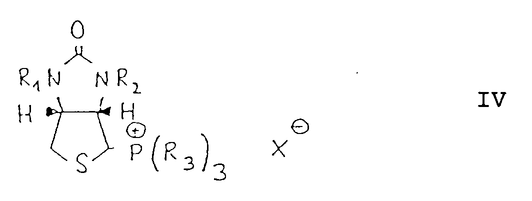 wherein R₁ represents an (R)- or (S)-1-phenyl-(C₂-C₄)-alkyl group, an (R)- or (S)-1-(C₁-C₄)-alkoxycarbonyl-1-phenylmethyl group, an (R)- or (S)-1-benzyloxycarbonyl-1-phenylmethyl group, or an (R)- or (S)-1-phenyloxycarbonyl-1-phenylmethyl group; R₂ represents hydrogen, a (C₁-C₄)-alkanoyl group, unsubstituted or substituted by halogen atoms, a benzoyl group or benzyl group, each unsubstituted or substituted by halogen atoms or (C₁-C₆)-alkyl or (C₁-C₆)-alkoxy groups, respectively, a (C₁-C₄)-alkoxycarbonyl group, a phenyl- or benzyloxycarbonyl group, a (C₁-C₄)-alkoxymethyl group, a pyranyl group, a benzenesulfonyl group, a p-toluenesulfonyl group, a (C₁-C₄)-alkylsulfonyl group, a diphenyl or dibenzylphosphinyl group, a di-(C₁-C₄)-alkoxyphosphinyl group or a tri-(C₁-C₄)-alkylsilyl group; R₃ represents an alkyl group having 1 - 20 C-atoms, a phenyl or benzyl group; and X represents a halogen atom, BF₄⁻, ClO₄⁻, J₃⁻ or PF₆⁻.

## Revendications

1. Procédé pour la préparation de l'ester de l'acide tétrahydro-1H-thiéno-[3,4-d]-imidazol-2(3H)-on-4-ylidenpentanoïque 1,3-substitué de la formule 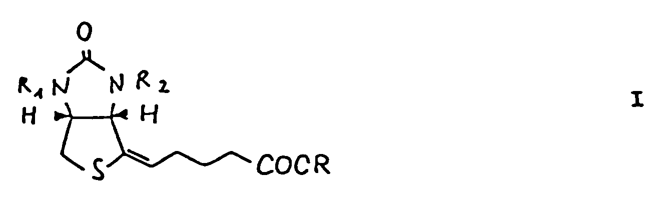 dans laquelle R = alkyle C₁-C₆ linéaire ou ramifié, non substitué ou substitué, ou un groupe phényle ou benzyle non substitué ou substitué par des atomes d'halogène, ou signifie des groupes alkyle ou aryle, et R₁ signifie un groupe phénylalkyle (R) ou (S)-1, un groupe (R) ou (S)-1-alcoxycarbonyl-1-phénylméthyle ou un groupe (R) ou (S)-1-aryloxycarbonyl-1-phénylméthyle et R₂ l'hydrogène, un groupe alcanoyle substitué ou non substitué, un groupe benzoyle ou un groupe benzyle, à chaque fois non substitué ou substitué, un groupe alcoxycarbonyle, un groupe aryloxycarbonyle, un groupe aryloxyalkyle, un groupe alcoxyalkyle, un groupe pyranyle, un groupe benzolsulfonyle, non substitué ou substitué, un groupe alkylsulfonyle, un groupe diarylphosphinyle, un groupe dialcoxyphosphinyle ou un groupe trialkylsilyle, caractérisé en ce que l'on réduit une tétrahydro-1H-thiéno-[3,4-d]-imidazol-2,4-dione 1,3-substituée de la formule 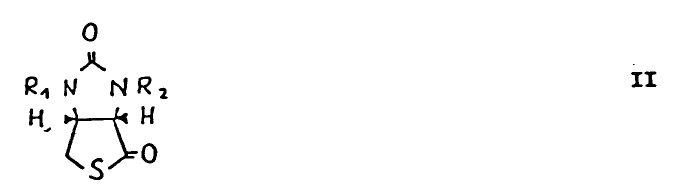 dans laquelle R₁ et R₂ ont la signification précitée, avec un agent de réduction en présence d'un solvant inerte en le composé hydroxy correspondant, en ce que l'on fait réagir celui-ci avec un sel de phosphonium de la formule
(R₃)₃PH^{⊕} X^{⊖} III
dans laquelle R₃ signifie un groupe alkyle avec 1 jusqu'à 20 atomes C, un groupe aryle ou benzyle et X signifie un atome d'halogène, BF₄⁻, ClO₄⁻, J₃⁻ ou PF₆⁻, en présence d'un solvant inerte dans un composé de phosphonium de la formule 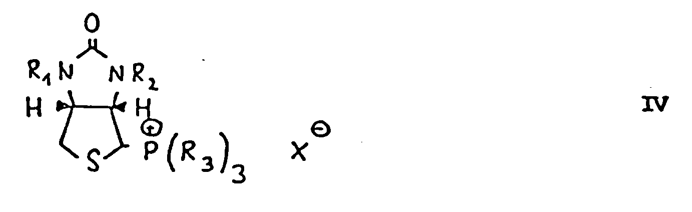 dans laquelle R₁, R₂, R₃ et X ont la signification précitée, et pour finir en ce que l'on transforme en présence d'une base forte et dans un solvant inerte avec un ester de l'acide 5-oxo-valérianique de la formule 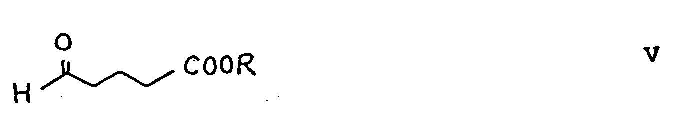 dans laquelle R a la signification précitée, en le produit final.

2. Procédé selon la revendication 1, caractérisé en ce qu'en tant qu'agent de réduction, on utilise un hydrure de diisobutylaluminium, un borhydrate de sodium, des complexes de bore ou des hydrures d'aluminium complexes.

3. Procédé selon la revendication 1, caractérisé en ce que l'on conduit la réaction avec de l'hydrure de diisobutylaluminium à des températures de -80 jusqu'à 25°C.

4. Procédé selon au moins l'une des revendications 1 à 3, caractérisé en ce que la formation du composé de phosphonium s'effectue à des températures de -20 à 100°C.

5. Procédé selon au moins l'une des revendications 1 à 4, caractérisé en ce qu'en tant que base forte pour la réaction avec l'ester de l'acide 5-oxo-valérianique, on utilise un alcoolat alcalin, un hexaméthyldisilazide alcalin, un hydrure alcalin ou un lithium alcalin.

6. Procédé selon au moins l'une des revendications 1 à 5, caractérisé en ce que la conversion s'effectue avec l'ester de l'acide 5-oxo-valérianique à des températures de -20 à 60°C.

7. Composés de phosphonium en tant que produits intermédiaires dans le procédé selon la revendication 1 de la formule 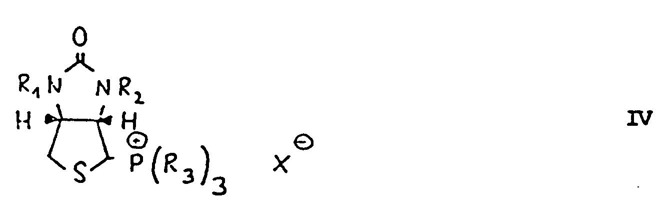 dans laquelle R₁ signifie un groupe (R) ou (S)-1-phényl-(C₂-C₄)-alkyle, un groupe (R) ou (S)-1-(C₁-C₄)-alcoxycarbonyl-1-phénylméthyle, un groupe (R) ou (S)-1-benzyloxycarbonyl-1-phénylméthyle ou un groupe (R) ou (S)-1-phényloxycarbonyl-1-phénylméthyle ; R₂ signifie l'hydrogène, un groupe alcanoyle (C₁-C₄) non substitué ou substitué, avec des atomes d'halogène ou un groupe benzyle ou benzoyle non substitué ou substitué par des atomes d'halogène ou par des groupes (C₁-C₆)-alkyle ou (C₁-C₆)-alcoxy, un groupe phényl- ou benzyloxycarbonyle, un groupe alcoxyméthyle (C₁-C₄), un groupe pyranyle, un groupe benzolsulfonyle, un groupe p-toluolsulfonyle, un groupe alkylsulfonyle en (C₁-C₄), un groupe diphényl- ou dibenzylphosphinyle, un groupe di(C₁-C₄)-alcoxyphosphinyle ou un groupe tri-(C₁-C₄)-alkylsilyle ; R₃ signifie un groupe alkyle avec 1-20 atomes C, un groupe phényle ou un groupe benzyle; et X signifie un atome d'halogène, BF₄⁻, ClO₄⁻, J₃⁻ ou PF₆⁻.
